# EUROPEAN PATENT APPLICATION

(11) **EP 4 637 275 A1**
(43) Date of publication of application: **22.10.2025**
(21) Application number: 23903004.2
(22) Date of filing: 05.07.2023
(51) Int. Cl.: H05H 7/10, H05H 13/04, A61N 5/10

(54) **ACCELERATOR ELECTROMAGNET, ACCELERATOR, AND PARTICLE BEAM THERAPY SYSTEM**

(30) Priority: 16.12.2022 JP 2022200988
(71) Applicant: HITACHI HIGH-TECH CORPORATION, Tokyo 105-6409 (JP)
(72) Inventor: TERADA Masanao, Tokyo 100-8280 (JP); EBINA Fuutarou, Tokyo 100-8280 (JP); NOMURA Takuya, Tokyo 100-8280 (JP)
(74) Representative: MERH-IP Matias Erny Reichl Hoffmann Patentanwälte PartG mbB
(86) International application number: PCT/JP2023/025031
(87) International publication number: WO 2024/127698

(57) **Abstract**

An electromagnet for accelerators includes a return yoke 42, and a pair of magnetic poles 41 fixed to the return yoke 42. The return yoke 42 has a return yoke hole 43 formed in an area intersecting an extraction orbit of a charged particle beam. The return yoke hole 43 is configured to give the hole 43 a different shape in accordance with an energy of the charged particle beam passing therethrough, thus adjusting a magnetic field distribution inside the return yoke hole 43. As a result, an electromagnet for accelerators, an accelerator including the electromagnet, and a particle beam therapy system that can reduce a gap between an orbit of high-energy charged particles and an orbit of low-energy charged particles to make the gap smaller than a gap in a conventional case.

## Description

### Technical Field

The present invention relates to an electromagnet for accelerators, an accelerator, and a particle beam therapy system.

### Background Art

Patent Literature 1 describes an example of an accelerator small in size and capable of extracting a beam carrying variable energy and a particle beam therapy system including the accelerator. The accelerator and particle beam therapy system include: an acceleration radiofrequency application device that is capable of frequency modulation and that applies an acceleration radiofrequency for accelerating a beam; a radiofrequency kicker that applies an extraction radiofrequency for extracting a beam, the extraction radiofrequency being different from the acceleration radiofrequency; a peeler magnetic field area and a regenerator magnetic field area that form a perturbation magnetic field area formed of a high-order magnetic field including a magnetic field component of two or more poles and including at least a quadrupole magnetic filed component; and a septum electromagnet having a magnetic material shim, an inner peripheral side septum coil conductor, an outer peripheral side septum coil conductor, a coil conductor connection, and a coil lead.

Patent Literature 2 describes a system according to which in a synchronous cyclotron, a correction coil is provided to cause charged particles carrying a low energy to approach an orbit of charged particles carrying a high energy, so that charged particles carrying different energies get on an extraction orbit at almost the same position.

### Citation List

### Patent Literature

PTL 1: JP 2020-38797 A
PTL 2: JP 2021-141062 A

### Summary of Invention

### Technical Problem

An accelerator is a device that accelerates charged particles, such as electrons, protons, and ions, to cause them to fly at high speed.

The accelerator is used for physical experiments in nuclei and elementary particles, radionuclide production, particle beam therapy, and the like. As an accelerator for particle beam therapy, in general, a circular accelerator is used.

Circular accelerators are roughly classified into two types. One type is a synchrotron that accelerates charged particles in such a way as to keep their orbit radius constant while adjusting a magnetic field and the frequency of an electric field that accelerates the particles, and the other type is a cyclotron that accelerates charged particles while increasing their orbit radius with a fixed magnetic field and electric field. One type of such a cyclotron is a synchronous cyclotron that adjusts the frequency of an electric field so that the electric field works in synchronization with acceleration of charged particles.

The synchrotron can extract charged particles carrying variable energy, whereas the cyclotron typically extracts charged particles carrying monochrome energy. The cyclotron can be made smaller than the synchrotron. However, the cyclotron is usually equipped with a device called degrader that after charged particles carrying monochrome energy are extracted, causes the charged particles to collide against each other to change their energy, which poses a problem that beam use efficiency drops.

A a solution to this problem, an idea of extracting charged particles carrying variable energy, using an accelerator like cyclotron that generates a fixed magnetic field, is described in Patent Literatures 1 and 2.

In a cyclotron type circular accelerator, the orbit radius of high-energy charged particles and that of low-energy charged particles are different in a fixed magnetic field, and consequently, a gap between an orbit of the high-energy charged particles and an orbit of the low-energy charged particles in the horizontal direction increases as these charged particles travel toward the downstream side of their orbits. The charged particles carrying the entire levels of energy that come out the accelerator are transported through a transport electromagnet to a target.

The transport electromagnet needs to let the entire charged particles coming out of the accelerator pass through a generated magnetic field space of the transport electromagnet, and to allow the entire charged particles to pass through the generated magnetic field space, the transport electromagnet must to be sizable enough. Meanwhile, it is desirable that the transport electromagnet be disposed close to a main electromagnet of the accelerator so that the charged particles suffer as less energy loss as possible.

In such circumstances, to prevent interference between the transport electromagnet and the main electromagnet, the transport electromagnet needs to be as small as possible. What is important to achieve this is to minimize the gap between the orbit of the high-energy charged particles and the orbit of the low-energy charged particles.

The present invention provides an electromagnet for accelerators, an accelerator including the electromagnet, and a particle beam therapy system that can reduce a gap between an orbit of high-energy charged particles and an orbit of low-energy charged particles to make the gap smaller than a gap in a conventional case.

### Solution to Problem

The present invention includes a plurality of means for solving the above problem. An example of such means is an electromagnet for an accelerator that generates charged particle beams carrying different energies. The electromagnet includes a return yoke, and a pair of magnetic poles fixed to the return yoke. The return yoke has a hole formed in an area intersecting an extraction orbit of the charged particle beam. The hole is configured to give the hole a different shape in accordance with an energy of the charged particle beam passing through the hole, thus adjusting a magnetic field distribution inside the hole.

### Advantageous Effects of Invention

According to the present invention, a gap between an orbit of high-energy charged particles and an orbit of low-energy charged particles can be reduced to make the gap smaller than a gap in a conventional case. Other problems, configurations, and effects that are not described above will be made clear by the following description of embodiments.

### Brief Description of Drawings

[FIG. 1] FIG. 1 is a bird's eye view of an accelerator according to a first embodiment.
[FIG. 2] FIG. 2 depicts a section of the accelerator according to the first embodiment.
[FIG. 3] FIG. 3 depicts a section of an extraction port of the accelerator according to the first embodiment.
[FIG. 4] FIG. 4 depicts a section of an extraction port of an accelerator according to a second embodiment.
[FIG. 5] FIG. 5 depicts a section of an extraction port of an accelerator according to a third embodiment.
[FIG. 6] FIG. 6 depicts a schematic configuration of a particle beam therapy system according to a fourth embodiment. Description of Embodiments

Hereinafter, embodiments of an electromagnet for accelerators, an accelerator, and a particle beam therapy system of the present invention will be described with reference to the drawings. In the drawings used in the present specification, the same or corresponding constituent elements are denoted by the same or similar reference signs, and repeated description of these constituent elements may be omitted.

What is described below is examples of implementation of the present invention, and is not intended to limit the content of the invention to specific modes described below. The invention can be implemented in various modes in accordance with the content of the invention described in the claims.

For example, in the following embodiment, an example of an eccentric orbit type accelerator, in which closed orbits of beams carrying different energies are eccentric from the center of the accelerator toward one side in the radial direction, will be described as an accelerator. The present invention, however, can be applied also to a different type of accelerator, such as a synchronous cyclotron, in which closed orbits of beams carrying different energies coincide with the center of the accelerator.

### [First Embodiment]

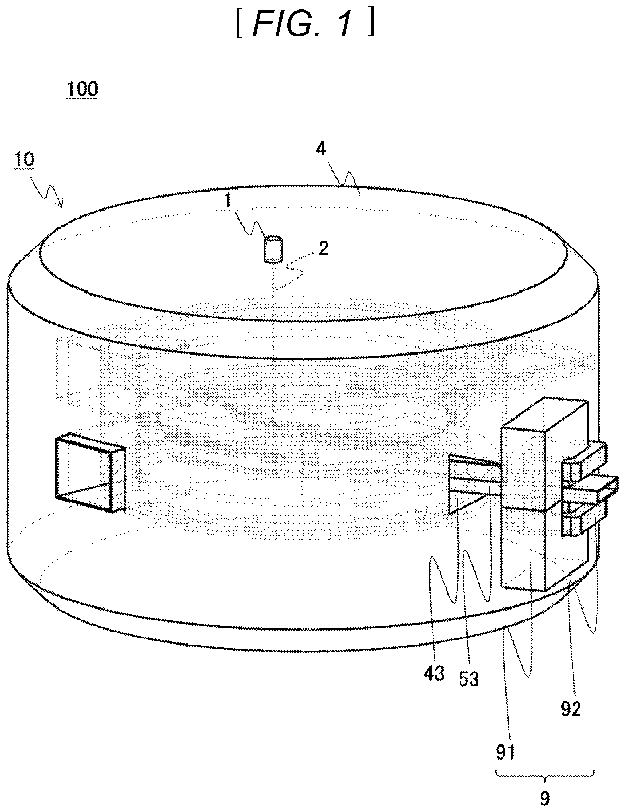

A first embodiment of an electromagnet for accelerators and an accelerator according to the present invention will be described with reference to FIGS. 1 to 3.

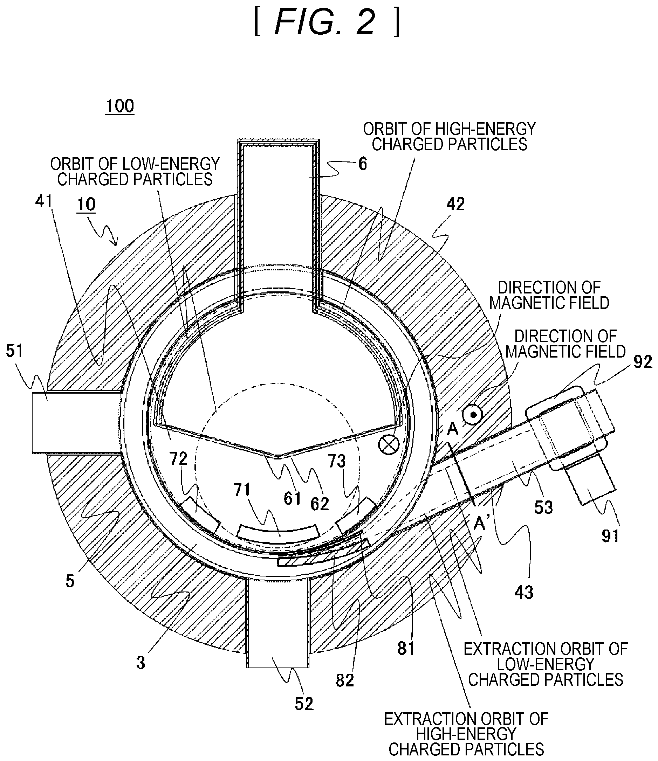

An overall configuration of an accelerator 100 including an electromagnet 10 for accelerators will first be described with reference to FIGS. 1 and 2. FIG. 1 is a bird's eye view of the accelerator according to the first embodiment, and FIG. 2 depicts a section of the accelerator according to the first embodiment.

The accelerator 100 shown in FIGS. 1 and 2 is an accelerator that generates charged particle beams carrying different energies and that is characterized by an eccentric orbit of charged particles. The accelerator 100 includes an injector 1, an incident charged particle introduction hole 2, a main electromagnet coil 3, a main electromagnet iron core 4, a vacuum container 5, a radiofrequency acceleration cavity 6, an extraction radiofrequency application device 71, an upstream-side multipolar magnetic field generation member 72, and a downstream-side multipolar magnetic field generation member 73 that are extraction adjustment equipment, a partition wall 81 and an antiseptum 82 that make up an extraction channel, a transport electromagnet iron core 91, and a transport electromagnet coil 92, which are shown in FIG. 1. The electromagnet 10 for accelerators has a return yoke 42, and a pair of magnetic poles 41 fixed to the return yoke 42.

A space where charged particles move is entirely covered with the vacuum container 5, and is connected to the outside of the accelerator 100 though a vacuuming port 51, a current extraction port 52, and an extraction port 53.

The injector 1 supplies charged particles to the incident charged particle introduction hole 2, thus injecting the charged particles into the accelerator through the incident charged particle introduction hole 2. Positively charged particles move counterclockwise in a circular orbit under the influence of a magnetic field (acing toward the far side of the paper surface) generated inside the main electromagnet coil 3 and the magnetic poles 41 of the main electromagnet iron core 4, and are accelerated by an accelerating electric field generated between a high-voltage electrode 61 and a ground electrode 62 by the radiofrequency acceleration cavity 6, thus gaining energy every time completing their orbit.

The charged particles having gained desired energy are then undergo the influence of an electric field generated by the extraction radiofrequency application device 71 and a multipolar magnetic field generated by the upstream-side multipolar magnetic field generation member 72 and the downstream-side multipolar magnetic field generation member 73, thus deviating from an eccentric orbit and getting on an extraction orbit.

The charged particles on the extraction orbit are led to the return yoke hole 43 formed on the return yoke 42 by a magnetic field generated between the partition wall 81 and the antiseptum 82. The return yoke hole 43 is a hole made in an area of return yoke 42 that intersects the extraction orbit of the charged particle beam.

The charged particles on the extraction orbit moves counterclockwise to reach the vicinity of the return yoke hole 43 under the influence of a magnetic field near the magnetic poles 41, and then undergoes a magnetic field opposite in direction to a main magnetic field inside the return yoke hole 43, thus orbiting clockwise.

The charged particles coming out of the return yoke hole 43 travels through the transport electromagnet 9 composed of the transport electromagnet iron core 91 and the transport electromagnet coil 92.

The magnetic field inside the return yoke hole 43 can be adjusted by the shape of the return yoke hole 43. Based on this fact, the following measures are taken to solve the problem mentioned above.

In a situation where charged particles experience the same magnetic field intensity, the orbit radius of low-energy charged particles is smaller than the orbit radius of high-energy charged particles. For this reason, a gap between the orbit of the low-energy charged particles and the orbit of the high-energy charged particles increases until these charged particles reach the return yoke hole 43. However, the gap between the orbit of the low-energy charged particles and the orbit of the high-energy charged particles decreases after these charged particles enter the return yoke hole 43. It is desirable that this gap be made as small as possible to improve efficiency in installation of the transport electromagnet 9 and to suppress an increase in the size of the transport electromagnet 9.

The return yoke hole 43 is configured to give the return yoke hole 43 a different shape in accordance with an energy of a charged particle beam passing therethrough, thus adjusting a magnetic field distribution inside the return yoke hole 43. For example, the return yoke hole 43 adjusts its internal magnetic field such that a magnetic field in an area where a charged particle beam on a low-energy side travels through is stronger than a magnetic field in an area where a charged particle beam on a high-energy side travels through.

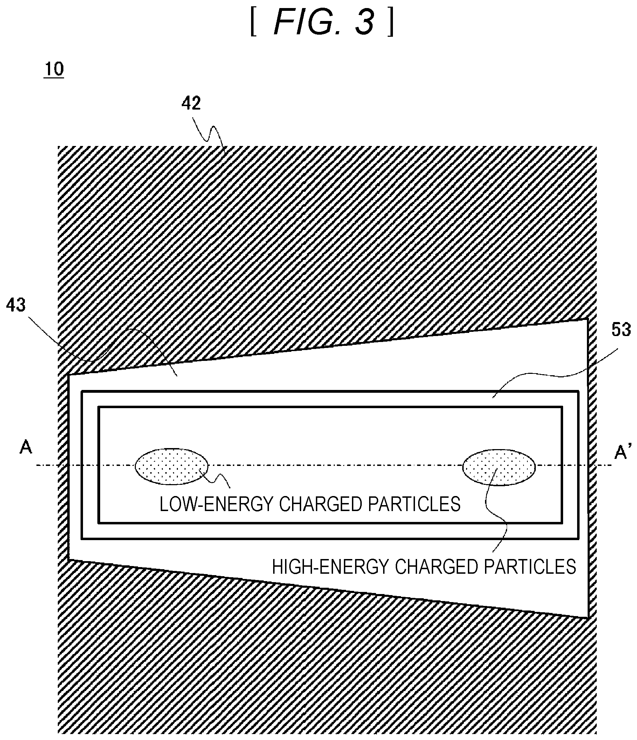

FIG. 3 shows an A-A' section of the return yoke hole 43 according to the embodiment of the present invention. The orbit of the low-energy charged particles is on an A side as the orbit of the high-energy charged particles is on an A' side.

For example, on the side of the orbit of the low-energy charged particles (low-energy side) inside the return yoke hole 43, a gap in the vertical direction (direction perpendicular to an A-A' line) of the return yoke hole 43 is reduced so that the magnetic field is increased to widely bend the orbit of the low-energy charged particles toward the orbit of the high-energy charged particles, as shown in FIG. 3. On the side of the orbit of the high-energy charged particles (high-energy side) inside the return yoke hole 43, on the other hand, a gap in the vertical direction of the return yoke hole 43 is made larger than the gap on the low-energy side so that a degree of bending of the orbit of the high-energy charged particles becomes smaller than a degree of bending of the low-energy charged particles.

In this manner, in the return yoke hole 43, the gap in the vertical direction on the low-energy side is made narrower than the gap in the vertical direction on the high-energy side.

The return yoke hole 43 has its wall surface in the circumferential direction of the accelerator formed into an inclined shape in which an end of the return yoke hole 43 on the low-energy side and an end of the return yoke hole 43 on the high-energy side are connected by straight lines.

The inclined shape of the return yoke hole 43 has upper and lower inclined shapes that are vertically symmetrical with each other.

With no particular limitations placed on the wall surface shape of the return yoke hole 43 in the transport direction of the charged particles, the wall surface shape can be made identical with the shape shown in FIG. 3 in all sides including the upstream side and downstream side in the transport direction. Also, the wall surface shape may be made different from the shape of FIG. 3 on the upstream side and/or the downstream side in the transportation direction. When the wall surface shape is changed on the upstream side and/or the downstream side in the transport direction, whether to narrow the gap on the low-energy side of the return yoke hole 43 to further strengthen the magnetic field or to widen the gap to weaken the magnetic field is decided depends on design, and the decision can be changed when necessary.

Effects of this embodiment will then be described.

The above-described electromagnet 10 for accelerators according to the first embodiment of the present invention is the electromagnet for the accelerator 100 that generates charged particle beams carrying different energies. The electromagnet includes the return yoke 42, and the pair of magnetic poles 41 fixed to the return yoke 42. The return yoke 42 has the return yoke hole 43 formed in the area intersecting the extraction orbit of the charged particle beam. The return yoke hole 43 is configured to give the return yoke hole 43 a different shape in accordance with an energy of the charged particle beam passing therethrough, thus adjusting the magnetic field distribution inside the return yoke hole 43. For example, the return yoke hole 43 adjusts its internal magnetic field such that the magnetic field in the area where the charged particle beam on the low-energy side travels through is stronger than the magnetic field in the area where charged particle beam on the high-energy side travels through.

This enhances an effect of bending the orbit of the low-energy charged particles, thus reducing the gap between the orbit of the low-energy charged particles and the orbit of the high-energy charged particles to make the gap smaller than the gap in the conventional case. Hence the gap between the orbit of the low-energy charged particles and the orbit of the high-energy charged particles can be reduced to be smaller than the gap in the conventional case. Thus, the return yoke hole shape of the main electromagnet for allowing extraction of charged particles carrying variable energy without equipment interference is achieved, and an increase in the size of the transport electromagnet 9 on the downstream side can be avoided.

In addition, having the gap in the vertical direction on the low-energy side that is narrower than the gap in the vertical direction on the high-energy side, the return yoke hole 43 allows creation of weak and strong magnetic fields through a simple shape. This further facilitates an improvement in efficiency in installation of the transport electromagnet 9 and suppression of an increase in the size of the transport electromagnet 9.

Furthermore, the return yoke hole 43 has its wall surface formed into inclined shapes in which the low-energy side and the high-energy side are connected by straight lines. Because of of this, in the accelerator 100 in which the energy of an extraction beam is changed continuously, the magnetic field intensity in the return yoke hole 43 is changed continuously as well. As a result, a proper magnetic filed design can be achieved.

### <Second Embodiment>

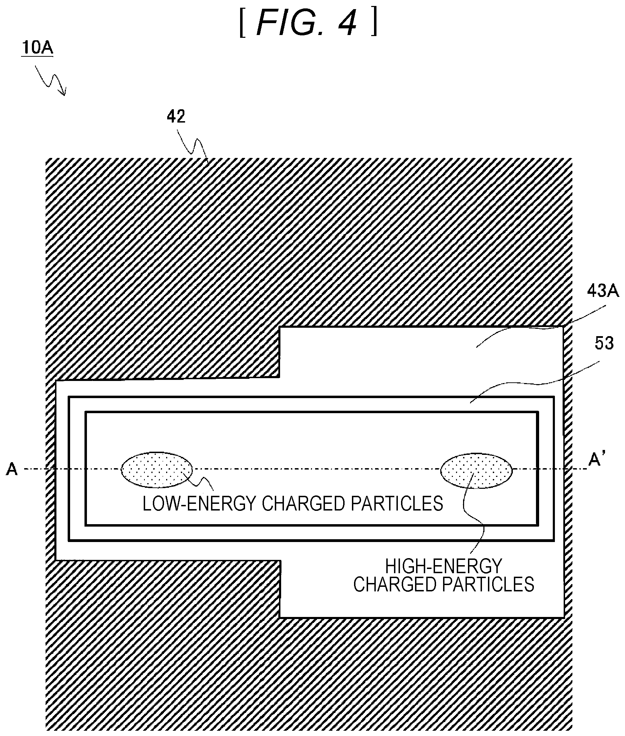

An electromagnet for accelerators and an accelerator according to a second embodiment of the present invention will be described with reference to FIG. 4. FIG. 4 depicts a section of an extraction port of the accelerator according to the second embodiment.

In an electromagnet 10A for accelerators of this embodiment shown in FIG. 4, a return yoke hole 43A formed on the return yoke 42 has its wall surface shape structured such that the low-energy side and the high-energy side are connected in a step shape, that is, the end of the return yoke hole 43 on the A side and the end of the return yoke hole 43 on the A' side are connected in a step shape. The number of steps of the step shape, which is not limited specifically, may be two or more.

Other configurations and operations are substantially the same as those of the electromagnet for accelerators and the accelerator according to the first embodiment described above, and therefore details of these configurations and operations will not be described further.

The electromagnet for accelerators and the accelerator according to the second embodiment of the present invention offers substantially the same effects as those of the electromagnet for accelerators and the accelerator according to the first embodiment described above.

Having the wall surface shape such that the low-energy side and the high-energy side are connected in the step shape, the return yoke hole 43A offers a structure effective for a case where a strong magnetic field should be created on the side of the orbit of energy charged particles, thus allowing an improvement in efficiency in installation of the transport electromagnet 9 and suppression of an increase in the size of the transport electromagnet 9 in the same manner as in the first embodiment.

### <Third Embodiment>

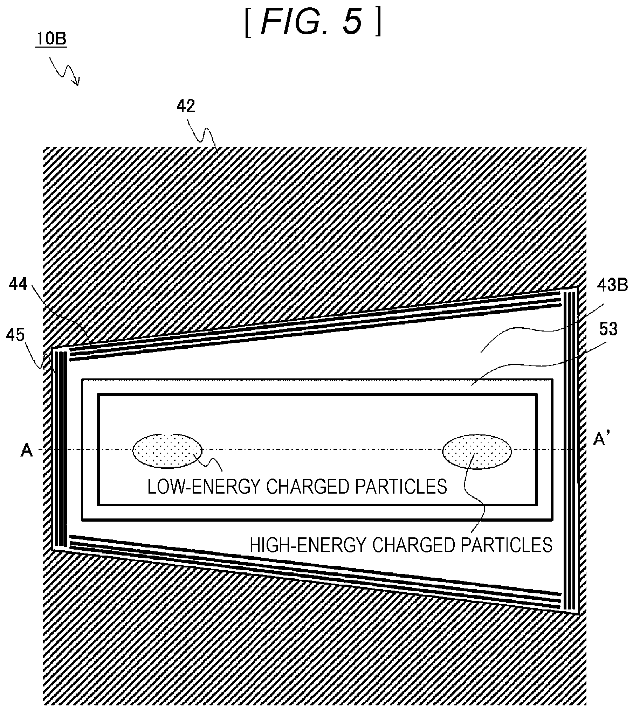

An electromagnet for accelerators and an accelerator according to a third embodiment of the present invention will be described with reference to FIG. 5. FIG. 5 depicts a section of an extraction port of the accelerator according to the third embodiment.

An electromagnet 10B for accelerators of the third embodiment shown in FIG. 5 has a return yoke hole 43B whose diameter is larger than the diameter of the return yoke hole 43 formed on the return yoke 42 of the electromagnet 10 for accelerators of the first embodiment. Inside the return yoke hole 43B is a mechanism that allows attachment and/or detachment of one or more horizontal iron plates 44 and/or vertical iron plates 45.

The form of the mechanism that allows attachment and/or detachment of the horizontal iron plate 44 and the vertical iron plate 45 is not specified in detail. The mechanism allow attachment and/or detachment of the horizontal and vertical iron plates by various means, such as bolting and sliding.

In addition, for example, when actual magnetic fields become uniformly larger than a design magnetic field, the actual magnetic fields can be lessened by adding another lateral iron plate 44 to reduce an area where magnetic flux penetrates. In another case where an actual magnetic field in an upper half side gets strong as an actual magnetic field in a lower half side gets weak, the vertical iron plates 45 on the upper side are reduced as the vertical iron plates 45 on the lower side are increased to adjust the gap in the vertical direction, which compensates the weakening field.

Other configurations and operations are substantially the same as those of the electromagnet for accelerators and the accelerator according to the first embodiment described above, and therefore details of these configurations and operations will not be described further.

The electromagnet for accelerators and the accelerator according to the third embodiment of the present invention offers substantially the same effects as those of the electromagnet for accelerators and the accelerator according to the first embodiment described above.

Inside the return yoke hole 43B, one or more horizontal iron plates 44 and vertical iron plates 45 can be attached and/or detached. As a result, a gap between magnetic fields resulting from an error in assembling electromagnets or a gap in magnetic fields caused by a magnetic material present nearby can be compensated, and therefore adjustment to a given magnetic field becomes more easy.

It should be noted that the inner periphery of the return yoke hole 43B, in which one or more horizontal iron plates 44 and vertical iron plates 45 are attached and/or detached, is not necessarily linearly shaped as shown in FIG. 5, but may be of the step shape of the second embodiment, as shown in FIG. 4.

### <Fourth Embodiment>

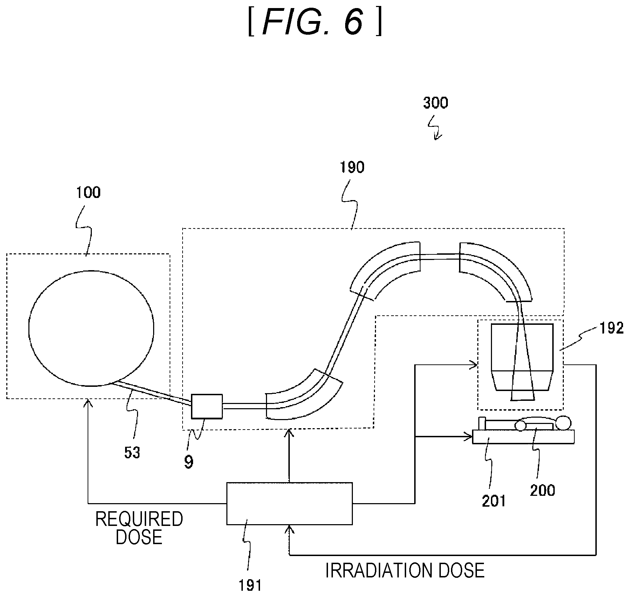

A fourth embodiment of the present invention in which the accelerator 100 of the above embodiments is applied to a particle beam therapy system used for particle beam therapy or the like will then be described with reference to FIG. 6. FIG. 6 depicts a schematic configuration of a particle beam therapy system according to the fourth embodiment.

A particle beam therapy system 300 shown in FIG. 6 includes the accelerator 100, the extraction port 53, a rotary gantry 190, an irradiation device 192, a treatment couch 201, and a controller 191.

An ion beam carrying a specific energy emitted from the accelerator 100 is transported to the irradiation device192 by the extraction port 53 and the rotary gantry 190. The transported ion beam carrying the specific energy is shaped by the irradiation device 192 to match the shape of an affected area, and a given does of the ion beam is emitted onto the target affected area of a patient 200 lying on the treatment couch 201.

Theses operations of the accelerator 100, the extraction port 53, the rotary gantry 190, the irradiation device 192, and the treatment couch 201 are executed by the controller 191.

The controller 191 is composed of a computer or the like. The computer making up the controller 191 includes a CPU, a memory, and an interface, and executes control of operation of each device and various arithmetic processes, which will be described later, based on various programs. These programs are stored in an internal recording medium, an external recording medium, or a data server in each constituent element, and are read and executed by the CPU.

Operation control processes may be integrated into one program, or distributed into a plurality of programs, or provided as a combination of one program and a plurality of programs. Some or all of programs may be run on dedicated hardware, or may be modularized. Furthermore, various programs may be delivered from a program distribution server, an internal storage medium, or an external recording medium and be installed in each device.

The electromagnet for accelerators incorporated in the accelerator 100 is not necessarily the electromagnet 10 for accelerators of the first embodiment, but may be either the electromagnet 10A for accelerators described in the second embodiment or the electromagnet 10B for accelerators described in the third embodiment, with no specific limitations applied thereto.

A plurality of irradiation devices 192 may be provided. The irradiation device 192 may be fixed, i.e., not allowed to rotate. An irradiation method carried out by the irradiation device 192 is not limited to a specific method. The irradiation device 192 may adopt either a scanning method of scanning with a beam or a wobbler method of using a scatterer.

### [Others]

It should be noted that the present invention is not limited to the above embodiments and that the invention includes various modifications. The above embodiments have been described in detail for easy understanding of the present invention, and are not necessarily limited to an embodiment including all constituent elements described above.

Some constituent elements of a certain embodiment may be replaced with constituent elements of another embodiment, and a constituent element of another embodiment may be added to a constituent element of a certain embodiment. In addition, some of constituent elements of each embodiment can be deleted therefrom or add to or replaced with constituent elements of another embodiment.

The embodiments of the present invention may be implemented in the following modes.
(1)An electromagnet for accelerators that generates charged particle beams carrying different energies is provided. The electromagnet includes a return yoke, and a pair of magnetic poles fixed to the return yoke. The return yoke has a hole formed in an area intersecting an extraction orbit of the charged particle beam. The hole is configured to give the hole a different shape in accordance with an energy of the charged particle beam passing through the hole, thus adjusting a magnetic field distribution inside the hole.
(2)In the electromagnet for accelerators described in (1), the hole adjusts its internal magnetic field such that a magnetic field in an area where the charged particle beam on a low-energy side travels through is stronger than a magnetic field in an area where the charged particle beam on a high-energy side travels through.
(3)In the electromagnet for accelerators described in (1), the gap has a structured in which a gap in the vertical direction on the low-energy side is narrower than a gap in the vertical direction on the high-energy side.
(4)In the electromagnet for accelerators described in (3), the hole has its wall surface formed into an inclined shape in which the low-energy side and the high-energy side are connected by straight lines.
(5)In the electromagnet for accelerators described in (3), the hole has its wall surface shape structured such that the low-energy side and the high-energy side are connected in a step shape.
(6)In the electromagnet for accelerators described in any one of (3) to (5), the hole allows one or more iron plates to be attached or detached inside the hole.
(7) (1) An accelerator including the electromagnet for accelerators described in any one of (1) to (6).
(8)A particle beam therapy system including the accelerator described in (7).

### Reference Signs List

- 1: injector
- 2: incident charged particle introduction hole
- 3: main electromagnetic coil
- 4: main electromagnet iron core
- 5: vacuum container
- 6: radiofrequency acceleration cavity
- 9: transport electromagnet
- 10, 10A, 10B: electromagnet for accelerators
- 41: magnetic pole
- 42: return yoke
- 43, 43A, 43B: return yoke hole (hole)
- 44: lateral iron plate (iron plate)
- 45: vertical iron plate (iron plate)
- 51: vacuuming port
- 52: current extraction port
- 53: extraction port
- 61: high-voltage electrode
- 62: ground electrode
- 71: extraction radiofrequency application device
- 72: upstream multipolar magnetic field generation member
- 73: downstream multipolar magnetic field generation member
- 81: partition wall
- 82: antiseptum
- 91: transport electromagnet iron core
- 92: transport electromagnet coil
- 100: accelerator
- 190: rotating gantry
- 191: controller
- 192: irradiation device
- 200: patient
- 201: treatment couch
- 300: particle beam therapy system

## Claims

1. An electromagnet for accelerators that generates charged particle beams carrying different energies, wherein
the electromagnet includes a return yoke, and a pair of magnetic poles fixed to the return yoke, wherein
the return yoke has a hole formed in an area intersecting an extraction orbit of each of the charged particle beams, and wherein
the hole is configured to give the hole a different shape in accordance with an energy of the charged particle beam passing through the hole, thus adjusting a magnetic field distribution inside the hole.

2. The electromagnet for accelerators according to claim 1,
wherein
the hole adjusts its internal magnetic field such that a magnetic field in an area where the charged particle beam on a low-energy side travels through is stronger than a magnetic field in an area where the charged particle beam on a high-energy side travels through.

3. The electromagnet for accelerators according to claim 2,
wherein
the hole has a structure in which a gap in a vertical direction on the low-energy side is narrower than a gap in a vertical direction on the high-energy side.

4. The electromagnet for accelerators according to claim 3,
wherein
the hole has its wall surface formed into an inclined shape in which the low-energy side and the high-energy side are connected by straight lines.

5. The electromagnet for accelerators according to claim 3,
wherein
the hole has its wall surface shape structured such that the low-energy side and the high-energy side are connected in a step shape.

6. The electromagnet for accelerators according to claim 3,
wherein
the hole allows one or more iron plates to be attached and/or detached inside the hole.

7. An accelerator comprising the electromagnet for accelerators according to any one of claims 1 to 6.

8. A particle beam therapy system comprising the accelerator according to claim 7.
